# EUROPÄISCHE PATENTSCHRIFT

(11) **EP 0 620 731 B1**
(45) Veröffentlichungstag und Bekanntmachung des Hinweises auf die Patenterteilung: **20.09.1995**
(21) Anmeldenummer: 93902180.4
(22) Anmeldetag: 12.01.1993
(51) Int. Cl.: A61K 9/46, A61K 9/16

(54) **PHARMAZEUTISCHE ZUBEREITUNG IN FORM EINER BRAUSE- UND/ODER ZERFALLSTABLETTE ODER EINES INSTANTGRANULATS, SOWIE VERFAHREN ZU IHRER HERSTELLUNG**
PHARMACEUTICAL PREPARATION IN THE FORM OF AN EFFERVESCENT AND/OR DISINTEGRATING TABLET OR AN INSTANT GRANULATE, AND PROCESS FOR PRODUCING IT
PREPARATION PHARMACEUTIQUE SOUS LA FORME D'UN COMPRIME EFFERVESCENT ET/OU A DESAGREGATION OU D'UN GRANULE INSTANTANE ET PROCEDE POUR SA FABRICATION

(30) Priorität: 13.01.1992 EP 92100441
(43) Veröffentlichungstag der Anmeldung: 26.10.1994
(73) Patentinhaber: Gergely, Gerhard, Dr., A-1053 Wien (AT)
(72) Erfinder: GERGELY, Gerhard, A-1053 Wien (AT); GERGELY, Thomas, A-1053 Wien (AT); GERGELY, Irmgard, A-1053 Wien (AT)
(74) Vertreter: Büchel, Kurt F., Dr.
(86) Internationale Anmeldenummer: EP9300055
(87) Internationale Veröffentlichungsnummer: WO9313760

(56) Entgegenhaltungen:
- EP-A- 0 103 387
- EP-A- 0 181 564
- EP-A- 0 208 144
- EP-A- 0 219 458
- EP-A- 0 393 909
- WO-A-87/01936
- GB-A- 2 160 100

## Beschreibung

Die Erfindung betrifft eine pharmazeutische Zubereitung in Form einer Brause- und/oder Zerfallstablette oder eines Instantgranulats, enthaltend wenigstens einen geschmacksirritierenden Wirkstoff und wenigstens eine die Freisetzung des Wirkstoffes verzögernde Matrix. Die Erfindung betrifft auch ein Verfahren zur Herstellung einer solchen pharmazeutischen Zubereitung.

Bisher war die Herstellung von Instant-Systemen, wie z.B. Brausetabletten und -granulaten, sowie löslichen Systemen auf geschmacksneutrale Wirkstoffe beschränkt. In zunehmendem Masse wird aber gefordert, auch solche Wirkstoffe in lösliche Instant-Systeme einzuarbeiten, die die Geschmacksnerven irritieren, insbesondere wenn ihr Geschmack sehr bitter ist. Beispiele dafür sind Dimenhydrinat, Codein, Loperamid, Diclophenac, Acelastin, Loperamidoxid, Domperidon, Cisaprid, Paracetamol und viele andere.

Erschwerend beim Einbau solcher Wirkstoffe ist ausserdem, dass bei Brausetabletten, die meistens ein Gewicht von 2-4 g haben, das Verhältnis von Wirkstoff zu Brausegranulat in eine Grössenordnung von bis zu 1:1000 gehen kann. Dies ergibt neben dem Problem der Bitternis ausserdem noch das schwer zu beherrschende Problem der Wirkstoff-Gleichverteilung.

Zur Maskierung eines zwar auch etwas bitter schmeckenden, im wesentlichen aber "kratzenden" Wirkstoffes, nämlich des Ibuprofens, wurde nun in der EP-B1-0181564 bereits vorgeschlagen, den Wirkstoff mit Fumarsäure und Hydrokolloiden zu umhüllen. In wässriger Lösung überdeckt der saure Geschmack den geringfügig bitteren; zu dieser Überdeckung tragen auch die Hydrokolloide etwas bei, sie dienen aber im wesentlichen als Suspensionshilfsmittel. Jedenfalls kann aber diese Umhüllung eine Freisetzung des Ibuprofens oder anderer, stärker geschmacksirritierender Wirkstoffe während einiger Minuten, während derer eine zubereitete Lösung steht, bevor sie getrunken wird, nicht hintanhalten.

Ausserdem hat man für Kautabletten, z.B.in der EP-A-212.641, schon vorgeschlagen, Dimenhydrinat - einen der genannten, geschmacksirritierenden Stoffe - durch Einbau in eine Polymetacrylsäureester-Matrix, z.B. in Eudragit S, zu maskieren, was vorgeblich auch gelingt. Allerdings wurde dabei nicht beachtet, dass Eudragit S im sauren Milieu schlecht löslich ist und daher den Wirkstoff erst im Darm freigibt, was für Dimenhydrinat (nur in Einzelfällen!) akzeptabel sein mag, nicht aber für Wirkstoffe, die im sauren Milieu des Magens freigesetzt werden sollen. Diese Lösung allein scheidet daher auch für z.B. Brausetabletten aus.

Das angestrebte Ziel wird erst erreicht, wenn die Matrix in inniger Mischung mit den wirkstoffteilchen vorliegt bzw. diese ummantelt und auf einem Träger aufgezogen ist, wobei die Zubereitung in wässriger Lösung bei Raumtemperatur innerhalb von etwa 2 min höchstens 65, vorzugsweise höchstens 50% des Wirkstoffs, jedoch innerhalb von maximal 20, vorzugsweise maximal 15 min in 0,1n HCl bei 38°C mehr als 70, vorzugsweise wenigstens 80% des Wirkstoffs freisetzt.

Damit sind die obgenannten Probleme erstmals überraschend in den Griff zu bekommen, insbesondere wenn die Matrix wenigstens einen Fettsäureester und/oder ein Wachs, vorzugweise mit einem Schmelzpunkt zwischen 30 und 45, insbesondere zwischen 32 und 35°C enthält. Sie kann zum Beispiel wenigstens ein Cellulosederivat, vorzugsweise Hydroxypropylmethylcellulose-Phthalat (HPMCP), Celluloseacetatphthalat (CAP), Cellulose-acetattrimellitat (CAT), oder Cellulose-acetatbutyrat (CAB), insbesondere wenigstens einen Polymetacrylsäureester, vorzugsweise Eudragit L allein oder in Mischung mit Eudragit S, enthalten.

Der Trägerstoff wird insbesondere aus wenigstens einer der Substanzen Mannit, Sorbit, Saccharose, Milchzucker, Aerosil, Stärke, Polyvinylpolypyrrolidon, oder auch aus einem oder mehreren Bestandteilen des Brausesystems, wie insbesondere einer essbaren, organischen Säure, beispielsweise Alginsäure, Zitronensäure, Weinsäure oder Adipinsäure, oder auch wenigstens einem Alkali- und/oder Erdalkalicarbonat und/oder -hydrogencarbonat gewählt.

Der - insbesondere geschmacksirritierende, und in organischen Lösungsmitteln bzw. Lösungsmittelgemischen, z.B. einem Alkohol, einem Keton oder einem chlorierten Kohlenwasserstoff lösliche - Wirkstoff wird in einer Menge von weniger als 60, vorzugsweise weniger als 10 mg, die Matrix in einer Menge des 1 bis 10-, vorzugsweise des 3 bis 5-fachen der Wirkstoffmenge pro Tablette oder Granulatdosis vorliegen.

Für einen Wirkstoff zur Behandlung von Diarrhöe, wie z. B. Loperamid, enthält die Zubereitung zusätzlich eine dem Ausgleich des Elektrolytverlustes im Körper dienende Mischung und Menge von Alkali- und/oder Erdalkalisalzen, vorzugsweise von organischen Salzen, sowie von Chloriden.

In dem erfindungsgemässen Verfahren wird der Wirkstoff mit der Matrix in einem gemeinsamen Lösungsmittel - vorzugsweise bei etwas erhöhter Temperatur - gelöst und die Lösung auf 10 bis 50, vorzugsweise auf 20 bis 40 Gewichtsteile (auf 1 Gewichtsteil der Summe von Wirkstoff und Matrix bezogen) eines neutralen Trägerstoffes gleichmässig aufgebracht und getrocknet. Anschliessend wird das erzielte Granulat mit den übrigen Brause- und/oder Zerfallskomponenten, sowie Aromen und dergleichen gemischt und gegebenenfalls zu Tabletten verpresst.

Die Erfindung bewährt sich insbesondere bei Zubereitungen mit sehr geringen Wirkstoffdosen, z.B. von weniger als 50, insbesondere weniger als 10 mg pro Tablette oder Sachet.

Das Verfahren beruht im wesentlichen darauf, dass der Wirkstoff vornehmlich in organischen Lösungsmitteln gemeinsam mit einem wasserunlöslichen oder in Wasser schwer löslichen Stoff, wie z.B. einem Cellulosederivat und/oder einem Fettsäureester bzw. Wachs, z.B. einem Glycerid gesättigter Fettsäuren, das gegebenenfalls vorher aufgeschmolzen wird, gelöst wird, was ohnedies vorzugsweise bei etwa 38 bis 40 °C geschieht. Diese Lösung wird dann auf einem Träger verteilt und das Lösungsmittel wird verdampft. Bei dem Träger kann es sich einerseits um neutrale Trägerstoffe, wie Mannit, Sorbit, Saccharose oder Lactose, andererseits aber auch um Aerosil oder Reisstärke handeln. Andererseits können aber auch einzelne Teile des Brausesystems oder dieses als ganzes als Träger fungieren, z.B. Alginsäure, Zitronensäure, Alkalibicarbonate oder -carbonate, etc.

Es findet sich sodann der Wirkstoff verteilungsfreundlich entweder auf dem Brausesystem, einem Teil davon, oder auf einem neutralen Träger, ist aber in einem Cellulosederivat und/oder in einem Glycerid gesättigter Fettsäuren eingebettet, das so formuliert ist, dass die Freisetzung des Wirkstoffes den erfindungsgemässen Bedingungen genügt, d.h. insbesondere zum grössten Teil erst bei erhöhter Temperatur im Magen freigegeben wird, oder in speziellen, gewünschten Fällen erst bei der Passage durch den Darmtrakt resorbiert werden kann.

Mögliche Matrix-Materialien - insbesondere in Kombination mit Fettsäureestern oder Wachsen - sind Celluloseacetatphthalat (CAP), Celluloseacetattrimellitat (CAT), Celluloseacetatbutyrat (CAB). Ideal im erfindungsgemässen Sinn ist jede Mischung, zum Beispiel auch von Weich- und Hartparaffinen, oder von entsprechenden synthetischen Produkten, die sich nach 2 min bei pH>3-4 und Raumtemperatur noch nicht oder nur wenig, bei pH<2-3 und 38°C nach 15 min jedoch rasch und weitgehend löst, gegebenenfalls auch ein Polymetacrylsäureester(gemisch).

Der Wirkstoff, der auf der Trägeroberfläche in der Matrix eingebettet und verteilt ist, wird nach dem Auflösen des Brausesystems und des Trägers in feinst verteilter Form suspendiert vorliegen. Liegt Aerosil als Träger vor, dann löst sich dieses zwar nicht im Brausesystem auf, jedoch ist durch die feinst verteilte, suspendierte, aber eingebettete Form während des Verlaufs des Auflösens und während des Trinkens eine Geschmacksmaskierung deutlich erreichbar, ohne dass die Partikel mit dem Wirkstoff am Glasrand haften. Man kommt infolge des grossen Adsorptionsvermögens auch mit wesentlich geringeren Gewichtsmengen aus - wie später in den Beispielen noch zu zeigen sein wird - als wenn z.B. Mannit od. dgl. als Träger fungieren.

Andererseits findet aber auch durch das erfindungsgemässe System keine der sonst üblichen Retardierungen von meist mehreren Stunden statt, sondern der Wirkstoff wird relativ rasch, d.h. beginnend, nur teilweise, frühestens nach 2 bis 4 min im Brausesystem, spätestens aber nach 10, äusserstenfalls 15 bis 20 Minuten, dann aber weitgehend, im Magen- oder Darmtrakt wieder freigesetzt. Dies grenzt die Erfindung deutlich gegen herkömmliche Retard-Produkte ab. Diese dienen dem Zweck, eine verzögerte Wirkstoff-Freigabe über mehrere Stunden zu erreichen, so dass mit einer einmaligen Dosierung ein längerer Wirkungszeitraum erreicht wird.

Der Einbau von Fettsäureestern geschieht vornehmlich bei Temperaturen, die über ihrem Schmelzpunkt liegen. Beispielsweise vermischt man die in organischen Lösungsmitteln gelösten Substanzen mit dem geschmolzenen Fettsäureglycerid und nimmt diesen verteilten Komplex in Aerosil auf. Man trocknet dann das Lösungsmittel ab und setzt das getrocknete, frei fliessende Produkt einer Brausemischung zu, oder aber man trägt es mitsamt dem Lösungsmittel auf die Brausemischung auf und verdampft es dort.

Dadurch erfolgt eine verstärkte Geschmacksmaskierung, aber auch eine beschleunigte Freigabe im Magen, da die Fettsäureglyceride aufschmelzen und den gesamten Komplex in der Wärme sofort freigeben.

Nun haben die meisten geschmacksirritierenden, insbesondere die bitteren Stoffe eine "Wahrnehmungsschwelle". Bei Loperamid beispielsweise wird ein halbes Milligramm noch nicht bitter empfunden, bei einem Milligramm ist die Bitternis noch erträglich, bei 2 mg wird sie für die meisten Personen bereits unerträglich.

Erfindungsgemäss werden nun Systeme vorgeschlagen, mit denen unmittelbar beim Brausen höchstens ein gerade noch erträglicher Teil des Wirkstoffes freigegeben wird; die Freigabe der restlichen Menge erfolgt aber dann sehr stark zeitverzögert, bzw. ergibt sie sich durch die im Magen- bzw. Darmtrakt herrschenden pH- und Temperaturverhältnisse.

### Beispiel 1:

Auch Dimenhydrinat lässt sich mit Gemischen von Eudragit L und Eudragit S in ähnlicher Weise verarbeiten. Um die Freisetzung allerdings nicht über Gebühr zu verzögern, kann man in diesem Fall wasserlösliche Substanzen mit einbauen, die das entstandene polymere Einschlussprodukt quasi perforieren. Dadurch kann der Wirkstoff leichter herausgelöst werden, noch bevor die Matrix selbst zur Gänze aufgelöst ist.

2 Gewichtsteile Dimenhydrinat werden mit 3 Gewichtsteilen Eudragit L und 3 Gewichtsteilen Eudragit S in einer Mischung von 35 Gewichtsteilen Alkohol und 10 Gewichtsteilen Isopropanol gelöst. In die Lösung werden fernerhin, je nach erwünschter Freigabe, 1 bis 3 Gewichtsteile Xylit oder Gluconsäure-Deltalacton mit aufgelöst. Sie wird auf 200 Gewichtsteile Sorbitol aufgebracht und bei 60°C - vorzugsweise unter Rühren im Vakuum - vom Lösungsmittel befreit.

Man bringt nun dieses Zwischenprodukt, das etwa 5 Gew.% Wirkstoff enthält, in ein Brausesystem ein, wobei die entstehende Suspensionsbrause trotz des eminent bitteren Geschmacks von Dimenhydrinat geschmacksneutral ist.

Um eine noch bessere Löslichkeit, bzw. Suspension des Wirkstoffes zu erzielen, kann man in die Lösung noch (ca. 10 bis 40 Gew.% der eingesetzten Menge Eudragit) Natriumchlorid, bzw. Gluconsäure-Deltalacton oder Zitronensäure einbringen, um das System durchlässiger zu gestalten. In speziellen Fällen ist es auch möglich, die Überzugsschicht mit Alkalicarbonaten bzw. -bicarbonaten zu dotieren, die mit Säuren reagieren, die Maskierung dadurch perforieren und durchlässiger machen.

### Beispiel 2:

Man geht wie in Beispiel 1 vor, nur werden 20 Gewichtsteile Dimenhydrinat mit 5 Gewichtsteilen Eudragit L und 50 Gewichtsteilen Witepsol H32 gemischt und auf 30 Gewichtsteile Aerosil aufgetragen. Die Freisetzung bei Raumtemperatur nach 2 min ist noch mehr verzögert, diejenige bei 38°C in künstlichem Magensaft beschleunigt.

### Beispiel 3:

Loperamid dient zur Behandlung von Diarrhöen. Bei Diarrhöen ist es indiziert, spezielle elektrolythaltige Flüssigkeiten gleichzeitig oder nachher mit einzunehmen, um den Elektrolyt-und Wasserverlust im Körper zu kompensieren. Der naheliegende Gedanke, Loperamid mit solchen Getränken zu vereinigen, scheiterte bisher aber an der extremen Bitternis des Wirkstoffes. Auch hier wurde gefunden, dass eine optimale Zuführung von Elektrolyten - wobei wesentlich die Zuführung von Natrium-, Kalium-, Calcium- und Magnesiumionen sowie auch von Natriumchlorid ist - über eine Elektrolyt-Brausetablette erfolgen kann, die bevorzugt folgendermassen zusammengesetzt ist:

2 Gewichtsteile Loperamid werden mit 1,5 Gewichtsteilen Eudragit L in einer Mischung aus gleichen Gewichtsteilen Alkohol, Aceton und Isopropanol bei 40°C gelöst und auf 200 Gewichtsteile Sorbit aufgebracht. Dies erfolgt vorzugsweise im Vakuum unter Rühren; anschliessend wird getrocknet. Das entstehende Granulat wird mit einem Brausesystem folgender Zusammensetzung gemischt:
153 Gew.teile Kaliumhydrogencarbonat (entsprechend 60 mg K)
50 Gew.teile Calciumcarbonat (entsprechend 20 mg Ca)
150 Gew.teile Natriumcarbonat (entsprechend 65 mg Na)
565 Gew.teile Natriumbicarbonat (entsprechend 220 mg Na)
165 Gew.teile Natriumchlorid (entsprechend 100 mg Cl)
91 Gew.teile Magnesiumoxyd (entsprechend 50 mg Mg)
2180 Gew.teile Zitronensäure
Die fertige Mischung kann noch mit geeigneten Süssstoffen und Aromen versehen und entweder in Sachets gefüllt oder zu Tabletten verpresst werden, die 2mg Loperamid pro Tablette enthalten. Ist letzteres vorgesehen, geht man zweckmässigerweise nach einem der in der CH-A-662926 oder der EP-A-272312 beschriebenen Verfahren vor, deren Inhalt im Rahmen der vorliegenden Beschreibung als geoffenbart gilt.

### Beispiel 4: Vergleich diverser Matrix- und Trägermaterialien:

In den folgenden Beispielen sind die Mengenangaben in mg pro Tablette zu verstehen. In allen Fällen werden 2 mg Loperamid gemeinsam mit dem Matrix-Material in 30 mg einer Mischung aus gleichen Teilen Alkohol, Aceton und Isopropanol bei etwa 40°C gelöst und auf den Träger aufgebracht. Dies erfolgt vorzugsweise im Vakuum unter Rühren; anschliessend wird getrocknet. Das entstehende Granulat wird mit einem Elektrolyt-Brausesystem folgender Zusammensetzung gemischt:
165 Natriumchlorid
149 Kaliumhydrogencarbonat
154 Calciumlactat
333 Magnesiumcitrat
722 Natriumhydrogencarbonat
500 Glucose
1900 Zitronensäure
Die Calcium- und Magnesiumsalze werden - da auch Glucose in der Tablette enthalten sein soll - aus Stabilitätsgründen in Form organischer Salze eingebaut.

Wenn in den vorangegangenen und den folgenden Beispielen von der Freisetzung nach 2 Minuten bzw. nach 15 Minuten die Rede ist, dann ist dies immer wie folgt zu verstehen:
2 min: 2 Tabletten werden in 200 ml H₂O von Raumtemperatur gelöst; nach 2 min werden 50 ml der Lösung mit 50 ml H₂O verdünnt und filtriert; das Filtrat wird auf den Gehalt an freigesetztem Wirkstoff (in %) untersucht
15 min: 2 Tabletten werden in 200 ml 0,1n HCl (künstlicher Magensaft) bei 38°C gelöst und 15 min auf dieser Temperatur gehalten; anschliessend werden 50 ml der Lösung mit 50 ml 0,1n HCL verdünnt und filtriert; das Filtrat wird auf den Gehalt an freigesetztem Wirkstoff (in %) untersucht.

### Negativbeispiele:

Es hat sich gezeigt, dass Eudragit S allein, das sich über pH 7 löst, oder HPMCP (Hydroxypropylmethylcellulose-Phthalat) zwar gut maskiert (Freisetzung nach 2 min nur 8.15% je nach Träger, dessen Art teilweise sehr unterschiedliche Ergebnisse bringt), aber im sauren Magensaft selbst nach 15 min zu wenig (12-50%) freisetzt; die Hauptmenge des Wirkstoffs wird erst im alkalischen Darmmilieu freigegeben.

Mischungen von Eudragit S mit Eudragit L und/oder HPMCP verhalten sich zwar etwas besser, aber immer noch ungenügend. Auch der Versuch, die Matrix von Eudragit S durch wechselnde Mengen löslicher Substanzen in der Matrix zu perforieren, um eine bessere Freigabe zu erhalten, bringt nicht den gewünschten Erfolg.

Eudragit E allein, das säurelöslich ist, setzt bereits nach 2 min 75-90% des Wirkstoffes frei und kommt daher nicht in Frage. Auch in Mischung mit anderen Eudragiten ist die Freisetzung nach 2 min noch immer zu hoch.

Eudragit L allein löst sich bei einem pH über 5,5; die Wirkung ist sehr mengenabhängig: 2-3 Gewichtsteile Eudragit L setzen auch nach 15 min nur 50 - 60 % frei.

### Positivbeispiele:

Eine wesentliche Verbesserung der Lösung der erfindungsgemäss gestellten Aufgabe lässt sich - wie bereits oben erwähnt - überraschend durch Fettsäureester, insbesondere durch Glyceride gesättigter Fettsäuren (Adeps solidus/Adeps neutralis, z.B. Witepsol H32 ^{(R)}, eingetragenes Warenzeichen der Firma Dynamit Nobel, im folgenden kurz "Wi 32") erzielen, die unter 45, vorzugsweise unter 35°C schmelzen, vorzugsweise in Kombination mit den vorerwähnten Matrixmaterialien, insbesondere von Cellulosederivaten, gegebenenfalls auch von Polymetacrylsäureestern wie z.B. Eudragit L (im folgenden kurz "Eu L"). Dabei wirken Fettsäureester oder Wachse bzw. Stearinsäure allein zwar auch schon in erfinderischem Sinn, aber noch nicht optimal. Sie müssen in grösseren Mengen eingesetzt werden, was für feste orale Darreichungsformen kein Problem darstellt. In wässriger Suspension neigen derart maskierte Wirkstoffteilchen zum Aufschwimmen oder Absinken, was man aber eventuell in den Griff bekommen kann.

Der Schmelzpunkt lässt sich übrigens bekanntlich auch durch Emulgatoren, wie z.B. Mygliol bzw. Lecithine erniedrigen. Diese wirken auch als Suspensionshilfsmittel, wie übrigens auch Propylenglykol (im folgenden kurz "Prop.glyk."), Polysorbat (Tween ^{(R)}), Alkylarylpolyglycolether (Eumulgin ^{(R)}) etc.

| Beispiel | Träger | Matrix | 2 min | 15 min |
|---|---|---|---|---|
| 4.0 | 200 Mannit | 10 Wi 32 | 50 | 90 |
| | | 0,1 DOSS | | |
| 4.1 | 200 Mannit | 7,5 Wi 32 | 40 | 92 |
| | | 2 HPMCP | | |
| 4.2 | 200 Mannit | 7,5 Wi 32 | 47 | 92 |
| | | 2 CAT | | |
| | | 0,375 Eumulgin | | |
| 4.3 | 200 Mannit | 7,5 Wi 32 | 39 | 100 |
| | | 2 Aerosil | | |
| | | 2 Eu L | | |
| 4.4 | 50 Reisstärke | 7,5 Wi 32 | 49 | 91 |
| | | 2 Eu L | | |
| 4.5 | 20 Aerosil | 7,5 Wi 32 | 47 | 98 |
| | | 2 Eu L | | |
| | | 0,075 Tween | | |
| 4.6 | 15 Aerosil | 7,5 Wi 32 | 62 | (*)94 |
| | 5 Reisstärke | 2 Eu L | | |
| 4.7 | 20 Aerosil | 5 Wi 32 | 50 | 100 |
| | | 3 Eu L | | |
| | | 0,05 Prop.glyk. | | |
| 4.8 | 50 Alginsäure | 5 Wi 32 | 36 | 86 |
| | | 3 Eu L | | |
| | | 0,05 Prop.glyk. | | |
| 4.9 | 100 Reisstärke | 5 Wi 32 | 33 | 93 |
| | | 3 Eu L | | |
| | | 0,05 Prop.glyk. | | |

| | | | | |
|---|---|---|---|---|
| (*) Zuerst das Aerosil in die Lösung einbringen, dann Reisstärke zufügen; schmeckt schon nur mehr ganz wenig bitter. | | | | |

| Beispiel | Träger | Matrix | 2 min | 15 min |
|---|---|---|---|---|
| 4.10 | 200 Saccharose | 5 Wi 32 | 38 | 85 |
| | | 1,5 Eu L | | |
| 4.11 | 200 Zitronensre. | 5 Wi 32 | 45 | 92 |
| | | 1,5 Eu L | | |
| 4.12 | 20 Aerosil | 5 Wi 32 | 60 | 96 |
| | | 3 HPMCP | | |
| 4.13 | 200 Sorbit | 1,5 Eu L | 55 | 70 |
| 4.14 | 3379 Elektrolytbrausesystem | - | - | - |

Wie aus den Beispielen 4.10 und 4.11 zu ersehen ist, kann bei relativ leicht löslichen Trägern, wie Saccharose oder Zitronensäure, die bei Aerosil als Träger verwendete Menge des Eudragit beträchtlich, nämlich auf die Hälfte reduziert werden, um dieselbe Freisetzung zu erzielen.

1,5 Eudragit L allein setzt nach 2 min 55 % frei und liegt damit an der obersten Toleranzgrenze; nach 15 min sind aber immer noch nur 70 % des Wirkstoffes freigesetzt,was einer unteren Toleranzgrenze entspricht.

Ganz ähnlich wie Witepsol ^{(R)} wirken hydriertes Ricinusöl (Cutina HR ^{(R)}, insbesondere für Zubereitungen ohne Brausesystem); Erdnussbutter (Oleum Arachidis hydrogenatum); Kakaobutter (vorwiegend Oleo-Palmito-Stearin); Cetaceum (Walrat = Palmitinsäureethylester); Glycerinmonodistearat (z.B. Imwitor ^{(R)} 900); Polyoxyethylenpalmitat; Triglyceride gemischter Fettsäuren oder Polyethylenglykole: jeweils 5 mg davon mit 3 mg Eudragit L auf 20 mg Aerosil setzen nach 2 min 40 - 60 %, nach 15 min 70 - 96 % des Wirkstoffes frei.

Wie aus Beispiel 4.14 ersichtlich, kann bei grosser Menge eines Elektrolytbrausesystems unter Umständen bei Loperamid auch auf die Maskierung verzichtet werden.

### Beispiel 5:

Diclophenac-Natrium ist sehr leicht wasserlöslich. Man löst 1 Gewichtsteil davon in 7,5 Gewichtsteilen Wasser auf und bringt die Lösung in einem Rühr- und Knetwerk bei 30 - 60°C auf 5 Gewichtsteile Alginsäure auf. Man fügt dann 0,3 Gewichtsteile Zitronensäure pulvis zu. Die Alginsäure wirkt hier sowohl als Träger wie auch - auf der Kornoberfläche - als Matrix; es entsteht ein frei fliessendes Pulver. Dieses wird - vorzugsweise im Vakuum - auf unter 1% Restfeuchtigkeit getrocknet, durch ein Sieb von 0,5 mm gesiebt und einer üblichen Brausemischung beigefügt.

Sollen beispielsweise zu 2 bis 4 g einer solchen Brausemischung 25 mg Diclophenac-Natrium beigefügt werden, so sind von dem erfindungsgemäss hergestellten, geschmacksneutralen Diclophenac-Komplex 157,5 mg nötig, eine Menge, die sich in einer gut konzipierten Brausemischung anstandslos gleichmässig verteilt. Sie schmeckt nach dem Auflösen neutral, während Diclophenac-Natrium einen extrem bitteren Geschmack aufweist.

### Beispiel 6:

Man geht wie in Beispiel 5 vor, nur werden 25 Gewichtsteile Diclophenac-Natrium mit je 5 Gewichtsteilen Eudragit L und Witepsol H32 in 50 Gewichtsteilen Äthanol und 20 Gewichtsteilen Wasser gelöst und auf 20 Gewichtsteile Aerosil aufgetragen, während anschliessend das Lösungsmittel verdampft wird. In Wasser ergibt sich eine schöne, nicht bitter schmeckende Lösung; die Sicherheit einer geringen Freisetzung nach 2 und einer höheren Freisetzung nach 15 Minuten hat sich verbessert.

### Beispiel 7:

Bei verschiedenen Zubereitungen von Codein, selbst zusammen mit anderen Wirkstoffen, ist sein bitterer Geschmack ausserordentlich störend. Theoretisch könnte Codein in derselben Weise wie das in Beispielen 5 und 6 erwähnte Diclophenac verarbeitet werden. Es hat sich jedoch hier ein anderes Vorgehen als zweckmässiger erwiesen:
1 Gewichtsteil Codein-Base wird mit 1 Gewichtsteil Eudragit S und 1 Gewichtsteil Eudragit L in 20 Gewichtsteilen Alkohol zur Lösung gebracht. Die Lösung bringt man auf 100 Gewichtsteile granuliertem Mannitol (mit einer Korngrösse von 0,2 bis 0,4 mm) auf und verdampft das Lösungsmittel, zwecks Verhinderung von Verteilungsunterschieden vorzugsweise unter Rühren im Vakuum. Das Endprodukt wird durch ein Maschensieb von 1 mm gesiebt, wobei für die spätere Dosierung 1 Gewichtsteil Codein-Base 13 Gewichtsteile der Mischung in eine Brausetablette eingesetzt werden müssen. Das Codein hat im Brausegetränk seine Bitternis völlig verloren und wird trotzdem nach 15 bis 20 Minuten bei 37 °C freigesetzt.

### Beispiel 8:

Man geht wie in Beispiel 7 vor, nur werden 26,6 Gewichtsteile 74%iges Codeinphosphat mit 5 Gewichtsteilen Eudragit L, 7,5 Gewichtsteilen Witepsol H32 und 2,5 Gewichtsteilen Polysorbat (zwecks besserer Suspension) in einem Gemisch von je 100 Gewichtsteilen Wasser, Äthanol, Aceton und Isopropanol gelöst und auf 20 Gewichtsteile Aerosil aufgetragen. Anschliessend wird das Lösungsmittel verdampft. Damit verbessert sich die Sicherheit einer geringen Freisetzung des Codeins nach 2 min bzw. einer hohen Freisetzung nach 15 min.

Aus den verschiedenen Beispielen ist zu ersehen, dass es sehr vom Wirkstoff und vom Träger abhängt, wieviel Matrix welcher Zusammensetzung man einsetzen muss, um die geeignete Maskierung bzw. Freisetzung zu erzielen.

## Patentansprüche (Patentansprüche für folgende(n) Vertragsstaat(en): AT, BE, CH, LI, DE, DK, FR, GB, GR, IE, IT, NL, PT, SE)

1. Pharmazeutische Zubereitung in Form einer Brause- und/oder Zerfallstablette oder eines Instantgranulats, enthaltend wenigstens einen geschmacksirritierenden Wirkstoff und wenigstens eine die Freisetzung des Wirkstoffes verzögernde Matrix, dadurch gekennzeichnet, dass die Matrix in inniger Mischung mit den Wirkstoffteilchen vorliegt bzw. diese ummantelt und auf einem Träger aufgezogen ist, wobei die Zubereitung in wässriger Lösung bei Raumtemperatur innerhalb von etwa 2 min höchstens 65, vorzugsweise höchstens 50% des Wirkstoffs, jedoch innerhalb von maximal 20, vorzugsweise maximal 15 min in 0,1n HCl bei 38°C mehr als 70, vorzugsweise wenigstens 80% des Wirkstoffs freisetzt.

2. Zubereitung nach Anspruch 1, dadurch gekennzeichnet, dass die Matrix wenigstens einen Fettsäureester und/oder ein Wachs, vorzugweise mit einem Schmelzpunkt zwischen 30 und 45, insbesondere zwischen 32 und 35°C enthält.

3. Zubereitung nach Anspruch 1 oder 2, dadurch gekennzeichnet, dass die Matrix wenigstens ein Cellulosederivat, vorzugsweise Hydroxypropylmethylcellulose-Phthalat (HPMCP), Celluloseacetatphthalat (CAP), Cellulose-acetattrimellitat (CAT), oder Cellulose-acetatbutyrat (CAB) enthält.

4. Zubereitung nach einem der vorhergehenden Ansprüche, dadurch gekennzeichnet, dass der Trägerstoff wenigstens eine der Substanzen Mannit, Sorbit, Saccharose, Milchzucker, Aerosil, Stärke, Polyvinylpolypyrrolidon, oder einen oder mehrere Bestandteile des Brausesystems umfasst, wie insbesondere eine essbare, organische Säure, beispielsweise Alginsäure, Zitronensäure, Weinsäure oder Adipinsäure, oder auch wenigstens ein Alkali- und/oder Erdalkalicarbonat und/oder -hydrogen-carbonat.

5. Zubereitung nach einem der vorhergehenden Ansprüche, dadurch gekennzeichnet, dass der Wirkstoff in einer Menge von weniger als 60, vorzugsweise weniger als 10 mg, die Matrix in einer Menge des 1 bis 10-, vorzugsweise des 3 bis 5-fachen der Wirkstoffmenge pro Tablette oder Granulatdosis vorliegen.

6. Zubereitung nach einem der vorhergehenden Ansprüche, für einen Wirkstoff zur Behandlung von Diarrhöe, wie z. B. Loperamid, dadurch gekennzeichnet, dass sie zusätzlich eine dem Ausgleich des Elektrolytverlustes im Körper dienende Mischung und Menge von Alkali- und/oder Erdalkalisalzen, vorzugsweise von organischen Salzen, sowie von Chloriden enthält.

7. Zubereitung nach einem der vorhergehenden Ansprüche, dadurch gekennzeichnet, dass die Matrix ausserdem wenigstens eine der Substanzen Gluconsäure-Deltalacton, Zitronensäure, Alkali- bzw. Erdalkalicarbonate bzw. -bicarbonate enthält.

8. Zubereitung nach einem der vorhergehenden Ansprüche, dadurch gekennzeichnet, dass sie wenigstens einen Polymetacrylsäureester, vorzugsweise Eudragit L allein oder in Mischung mit Eudragit S, enthält.

9. Verfahren zur Herstellung einer Zubereitung nach einem der vorhergehenden Ansprüche, dadurch gekennzeichnet, dass der Wirkstoff mit der Matrix in einem gemeinsamen Lösungsmittel - vorzugsweise bei etwas erhöhter Temperatur - gelöst, die Lösung auf 10 bis 50, vorzugsweise auf 20 bis 40 Gewichtsteile (auf 1 Gewichtsteil der Summe von Wirkstoff und Matrix bezogen) eines neutralen Trägerstoffes gleichmässig aufgebracht und getrocknet wird, worauf das erzielte Granulat mit den übrigen Brause- und/oder Zerfallskomponenten sowie Aromen und dergleichen gemischt und gegebenenfalls zu Tabletten verpresst wird.

## Patentansprüche (Patentansprüche für folgende(n) Vertragsstaat(en): ES)

1. Verfahren zur Herstellung einer pharmazeutischen Zubereitung in Form einer Brause- und/oder Zerfallstablette oder eines Instantgranulats, die wenigstens einen geschmacksirritierenden Wirkstoff und wenigstens eine die Freisetzung des Wirkstoffes verzögernde Matrix enthält, dadurch gekennzeichnet, dass die Matrix mit den Wirkstoffteilchen innig gemischt bzw. auf diese als Mantelschicht aufgezogen und schliesslich auf einem Träger aufgebracht wird, so dass die Zubereitung in wässriger Lösung bei Raumtemperatur innerhalb von etwa 2 min höchstens 65, vorzugsweise höchstens 50% des Wirkstoffs, jedoch innerhalb von maximal 20, vorzugsweise maximal 15 min in 0,1n HCl bei 38°C mehr als 70, vorzugsweise wenigstens 80% des Wirkstoffs freisetzt.

2. Verfahren nach Anspruch 1, dadurch gekennzeichnet, dass die Matrix wenigstens einen Fettsäureester und/oder ein Wachs, vorzugsweise mit einem Schmelzpunkt zwischen 30 und 45, insbesondere zwischen 32 und 35°C enthält.

3. Verfahren nach Anspruch 1 oder 2, dadurch gekennzeichnet, dass die Matrix wenigstens ein Cellulosederivat, vorzugsweise Hydroxypropylmethylcellulose-Phthalat (HPMCP), Celluloseacetatphthalat (CAP), Cellulose-acetattrimellitat (CAT), oder Cellulose-acetatbutyrat (GAB) enthält.

4. Verfahren nach einem der vorhergehenden Ansprüche, dadurch gekennzeichnet, dass der Trägerstoff wenigstens eine der Substanzen Mannit, Sorbit, Saccharose, Milchzucker, Aerosil, Stärke, Polyvinylpolypyrrolidon, oder einen oder mehrere Bestandteile des Brausesystems umfasst, wie insbesondere eine essbare, organische Säure, beispielsweise Alginsäure, Zitronensäure, Weinsäure oder Adipinsäure, oder auch wenigstens ein Alkali- und/oder Erdalkalicarbonat und/oder -hydrogen-carbonat.

5. Verfahren nach einem der vorhergehenden Ansprüche, dadurch gekennzeichnet, dass der Wirkstoff in einer Menge von weniger als 60, vorzugsweise weniger als 10 mg pro Tablette oder Granulatdosis, mit der Matrix in einer Menge des 1 bis 10-, vorzugsweise des 3 bis 5-fachen der Wirkstoffmenge gemischt bzw. ummantelt wird.

6. Verfahren nach einem der vorhergehenden Ansprüche, für einen Wirkstoff zur Behandlung von Diarrhöe, wie z.B. Loperamid, dadurch gekennzeichnet, dass zusätzlich eine dem Ausgleich des Elektrolytverlustes im Körper dienende Mischung und Menge von Alkali- und/oder Erdalkalisalzen, vorzugsweise von organischen Salzen, sowie von Chloriden zugesetzt werden.

7. Verfahren nach einem der vorhergehenden Ansprüche, dadurch gekennzeichnet, dass die Matrix ausserdem wenigstens eine der Substanzen Gluconsäure-Deltalacton, Zitronensäure, Alkali- bzw. Erdalkalicarbonate bzw. -bicarbonate enthält.

8. Verfahren nach einem der vorhergehenden Ansprüche, dadurch gekennzeichnet, dass wenigstens ein Polymetacrylsäureester, vorzugsweise Eudragit L allein oder in Mischung mit Eudragit S, zugesetzt wird.

9. Verfahren nach einem der vorhergehenden Ansprüche, dadurch gekennzeichnet, dass der Wirkstoff mit der Matrix in einem gemeinsamen Lösungsmittel - vorzugsweise bei etwas erhöhter Temperatur - gelöst, die Lösung auf 10 bis 50, vorzugsweise auf 20 bis 40 Gewichtsteile (auf 1 Gewichtsteil der Summe von Wirkstoff und Matrix bezogen) eines neutralen Trägerstoffes gleichmässig aufgebracht und getrocknet wird, worauf das erzielte Granulat mit den übrigen Brause- und/oder Zerfallskomponenten sowie Aromen und dergleichen gemischt und gegebenenfalls zu Tabletten verpresst wird.

## Claims (Claims for the following Contracting State(s): AT, BE, CH, LI, DE, DK, FR, GB, GR, IE, IT, NL, PT, SE)

1. Pharmaceutical formulation in the form of an effervescent and/or disintegrating tablet or of instant granules, containing at least one active ingredient having an irritating taste and at least one matrix which delays the release of the active ingredient, characterised in that the matrix is present as an intimate mixture with the active ingredient particles or covers said particles and is applied to a carrier, the formulation releasing at most 65%, preferably at most 50%, of the active ingredient in aqueous solution at room temperature within about 2 min but more than 70%, preferably at least 80%, of the active ingredient within max. 20 min, preferably max. 15 min, in 0.1 N HCl at 38°C.

2. Formulation according to Claim 1, characterised in that the matrix contains at least one fatty ester and/or one wax, preferably having a melting point between 30 and 45°C, in particular between 32 and 35°C.

3. Formulation according to Claim 1 or 2, characterised in that the matrix contains at least one cellulose derivative, preferably hydroxypropylmethylcellulose phthalate (HPMCP), cellulose acetate phthalate (CAP), cellulose acetate trimellitate (CAT) or cellulose acetobutyrate (CAB).

4. Formulation according to any of the preceding Claims, characterised in that the carrier comprises at least one of the substances mannitol, sorbitol, sucrose, lactose, Aerosil, starch and polyvinylpyrrolidone, or one or more components of the effervescent system, such as, in particular, an edible, organic acid, for example alginic acid, citric acid, tartaric acid or adipic acid, or at least one alkali metal and/or alkaline earth metal carbonate and/or bicarbonate.

5. Formulation according to any of the preceding Claims, characterised in that the active ingredient is present in an amount of less than 60 mg, preferably less than 10 mg, and the matrix is present in an amount of 1 to 10, preferably 3 to 5, times the amount of active ingredient per tablet or granule dose.

6. Formulation according to any of the preceding Claims, for an active ingredient for the treatment of diarrhoea, such as, for example, loperamide, characterised in that it additionally contains a mixture which serves to compensate the electrolyte loss in the body and an amount of alkali metal and/or alkaline earth metal salts, preferably of organic salts, and of chlorides.

7. Formulation according to any of the preceding Claims, characterised in that the matrix furthermore contains at least one of the substances gluconic acid delta-lactone, citric acid or alkali metal or alkaline earth metal carbonates or bicarbonates.

8. Formulation according to any of the preceding Claims, characterised in that it contains at leapt one polymethacrylate, preferably Eudragit L alone or as a mixture with Eudragit S.

9. Process for the preparation of a formulation according to any of the preceding Claims, characterised in that the active ingredient is dissolved with the matrix in a common solvent, preferably at slightly elevated temperature, the solution is uniformly applied to 10-50, preferably to 20-40, parts by weight (based on 1 part by weight of the sum of the active ingredient and matrix) of a neutral carrier and is dried, whereupon the granules obtained are mixed with the remaining effervescent and/or disintegration components and flavours and the like and, if required, are pressed to give tablets.

## Claims (Claims for the following Contracting State(s): ES)

1. Process for the manufacture of a pharmaceutical formulation in the form of an effervescent and/or disintegrating tablet or of instant granules, containing at least one active ingredient having an irritating taste and at least one matrix which delays the release of the active ingredient, characterised in that the matrix is intimately mixed with the active ingredient particles - or said particles are covered with it - and is applied to a carrier, the formulation releasing at most 65%, preferably at most 50%, of the active ingredient in aqueous solution at room temperature whithin about 2 min but more than 70%, preferably at least 80%, of the active ingredient within max. 20 min, preferably max. 15 min, in 0.1 N HCl at 38°C.

2. Process according to Claim 1, characterised in that the matrix contains at least one fatty ester and/or one wax, preferably having a melting point between 30 and 45°C, in particular between 32 and 35°C.

3. Process according to Claim 1 or 2, characterised in that the matrix contains at least one cellulose derivative, preferably hydroxypropylmethyl-cellulose phthalate (HPMCP), cellulose acetate phthalate (CAP), cellulose acetate trimellitate (CAT) or cellulose acetobutyrate (CAB).

4. Process according to any of the preceding Claims, characterised in that the carrier comprises at least one of the substances mannitol, sorbitol, sucrose, lactose, Aerosil, starch and polyvinylpyrrolidone, or one or more components of the effervescent system, such as, in particular, an edible, organic acid, for example alginic acid, citric acid, tartaric acid or adipic acid, or at least one alkali metal and/or alkaline earth metal carbonate and/or bicarbonate.

5. Process according to any of the preceding Claims, characterised in that the active ingredient is mixed - in an amount of less than 60 mg, preferably less than 10 mg - with the matrix in an amount of 1 to 10, preferably 3 to 5, times the amount of active ingredient per tablet or granule dose.

6. Process according to any of the preceding Claims, for an active ingredient for the treatment of diarrhoea, such as, for example, loperamide, characterised in that additionally a mixture is added which serves to compensate the electrolyte loss in the body and an amount of alkali metal and/or alkaline earth metal salts, preferably of organic salts, and of chlorides.

7. Process according to any of the preceding Claims, characterised in that the matrix furthermore contains at least one of the substances gluconic acid delta-lactone, citric acid or alkali metal or alkaline earth metal carbonates or bicarbonates.

8. Process according to any of the preceding Claims, characterised in that at least one polymethacrylate, preferably Eudragit L alone or as a mixture with Eudragit S is added.

9. Process according to any of the preceding Claims, characterised in that the active ingredient is dissolved with the matrix in a common solvent, preferably at slightly elevated temperature, the solution is uniformly applied to 10-50, preferably to 20-40, parts by weight (based on 1 part by weight of the sum of the active ingredient and matrix) of a neutral carrier and is dried, whereupon the granules obtained are mixed with the remaining effervescent and/or disintegration components and flavours and the like and, if required, are pressed to give tablets.

## Revendications (Revendications pour l'(les) Etat(s) contractant(s) suivant(s): AT, BE, CH, LI, DE, DK, FR, GB, GR, IE, IT, NL, PT, SE)

1. Préparation pharmaceutique sous la forme d'un granulé effervescent et/ou désintégrable ou d'un granulé instantané, contenant au moins une substance au goût irritant et au moins une matrice retardant la libération de la substance active, caractérisée en ce que la matrice est présente en mélange intime avec les particules de substance active ou les enveloppe et est fixée sur un support, la préparation libérant en solution aqueuse, à la température ambiante, sur une durée d'environ 2 minutes, au plus 65, de préférence au plus 50 % de la substance active, mais libérant sur une durée de 20 minutes au maximum, de préférence de 15 minutes au maximum, dans HCl 0,1N à 38°C, plus de 70 %, de préférence au moins 80 % de la substance active.

2. Préparation selon la revendication 1, caractérisée en ce que la matrice contient au moins un ester d'acide gras et/ou une cire ayant de préférence un point de fusion compris entre 30 et 45°C, en particulier entre 32 et 35°C.

3. Préparation selon les revendications 1 ou 2, caractérisée en ce que la matrice contient au moins un dérivé de la cellulose, de préférence du phtalate d'hydroxypropylméthyl cellulose (HPMCP), de l'acétate-phtalate de cellulose (CAP), de l'acétatetrimellitate de cellulose (CAT) ou de l'acétatebutyrate de cellulose (CAB).

4. Préparation selon l'une des revendications précédentes, caractérisée en ce que la substance de support comprend au moins une des substances suivantes : mannitol, sorbitol, saccharose, lactose, Aérosil, amidon, polyvinylpolypyrrolidone, ou un ou plusieurs constituants du système effervescent tels qu'en particulier un acide organique comestible, par exemple l'acide alginique, l'acide citrique, l'acide tartrique ou l'acide adipique, ou encore au moins un carbonate et/ou bicarbonate de métal alcalin et/ou alcalino-terreux.

5. Préparation selon l'une des revendications précédentes, caractérisée en ce que la substance active est présente dans une quantité inférieure à 60 ma, de préférence inférieure à 10 mg, la matrice dans une quantité égale à 1 à 10 fois, de préférence égale à 3 à 5 fois la quantité de substance active par comprimé ou dose de granulé.

6. Préparation selon l'une des revendications précédentes, pour une substance active destinée au traitement de la diarrhée telle que par exemple le Loperamid, caractérisée en ce qu'elle contient en outre un mélange servant à la compensation de la perte d'électrolyte dans le corps et une quantité de sels alcalins ou alcalino-terreux, de préférence de sels organiques, ainsi que de chlorures.

7. Préparation selon l'une des revendications précédentes, caractérisée en ce que la matrice contient en outre au moins une des substances suivantes : delta-lactone de l'acide gluconique, acide citrique, carbonates ou bicarbonates alcalins ou alcalino-terreux.

8. Préparation selon l'une des revendications précédentes, caractérisée en ce qu'elle contient au moins un ester d'acide polyméthacrylique, de préférence l'Eudragit L seul ou en mélange avec de l'Eudragit S.

9. Procédé de confection d'une préparation selon l'une des revendications précédentes, caractérisé en ce qu'on dissout la substance active avec la matrice dans un solvant commun, de préférence en chauffant légèrement, en ce qu'on applique régulièrement la solution sur 10 à 50 parties en poids, de préférence sur 20 à 40 parties en poids (pour une partie en poids de la somme de la substance active et de la matrice) d'une substance de support neutre et en ce qu'on la sèche, après quoi on mélange le granulé obtenu avec les autres constituants d'effervescence et de désintégration ainsi que des arômes etc., et le cas échéant on le presse à l'état de comprimés.

## Revendications (Revendications pour l'(les) Etat(s) contractant(s) suivant(s): ES)

1. Procédé de confection d' une préparation pharmaceutique sous la forme d'un granulé effervescent et/ou désintégrable ou d'un granulé instantané, contenant au moins une substance au goût irritant et au moins une matrice retardant la libération de la substance active, caractérisée en ce que la matrice est mélangée intimement avec les particules de substance active - ou les dites particules sont enveloppées de la dite substance - et est fixée sur un support, la préparation libérant en solution aqueuse, à la température ambiante, sur une durée d'environ 2 minutes, au plus 65, de préférence au plus 50 % de la substance active, mais libérant sur une durée de 20 minutes au maximum, de préférence de 15 minutes au maximum, dans HCl 0,1N à 38°C, plus de 70 %, de préférence au moins 80 % de la substance active.

2. Procédé selon la revendication 1, caractérisée en ce que la matrice contient au moins un ester d'acide gras et/ou une cire ayant de préférence un point de fusion compris entre 30 et 45°C, en particulier entre 32 et 35°C.

3. Procédé selon les revendications 1 ou 2, caractérisée en ce que la matrice contient au moins un dérivé de la cellulose, de préférence du phtalate d'hydroxypropylméthyl cellulose (HPMCP), de l'acétate-phtalate de cellulose (CAP), de l'acétate-trimellitate de cellulose (CAT) ou de l'acétate-butyrate de cellulose (CAB).

4. Procédé selon l'une des revendications précédentes, caractérisée en ce que la substance de support comprend au moins une des substances suivantes: mannitol, sorbitol, saccarose, lactose, Aérosil, amidon, polyvinylpolypyrrolidone, ou un ou plusieurs constituants du système effervescent tels qu'en particulier un acide organique comestible, par exemple l'acide alginique, l'acide citrique, l'acide tartrique ou l'acide adipique, ou encore au moins un carbonate et/ou bicarbonate de métal alcalin et/ou alcalino-terreux.

5. Procédé selon l'une des revendications précédentes, caractérisée en ce que la substance active est melangée - dans une quantité inférieure à 60 mg, de préférence inférieure à 10 mg - avec la matrice dans une quantité égale à 1 à 10 fois, de préférence égale à 3 à 5 fois la quantité de substance active par comprimé ou dose de granulé.

6. Procédé selon l'une des revendications précédentes, pour une substance active destinée au traitement de la diarrhée telle que par exemple le Loperamid, caractérisée en ce qu'on ajoute en outre un mélange servant à la compensation de la perte d'électrolyte dans le corps et une quantité de sels alcalins ou alcalino-terreux, de préférence de sels organiques, ainsi que de chlorures.

7. Procédé selon l'une des revendications précédentes, caractérisée en ce que la matrice contient en outre au moins une des substances suivantes: delta-lactone de l'acide gluconique, acide citrique, carbonates ou bicarbonates alcalins ou alcalino-terreux.

8. Procédé selon l'une des revendications précédentes, caractérisée en ce qu'on ajoute au moins un ester d'acide polyméthacrylique, de préférence l'Eudragit L seul ou en mélange avec de l'Eudragit S.

9. Procédé selon l'une des revendications précédentes, caractérisé en ce qu'on dissout la substance active avec la matrice dans un solvant commun, de préférence en chauffant légèrement, en ce qu'on applique régulièrement la solution sur 10 à 50 parties en poids, de préférence sur 20 à 40 parties en poids (pour une partie en poids de la somme de la substance active et de la matrice) d'une substance de support neutre et en ce qu'on la sèche, après quoi on mélange le granulé obtenu avec les autres constituants d'effervescence et de désintégration ainsi que des arômes etc., et le cas échéant on le presse à l'état de comprimés.
